Europäisches Patentamt

European Patent Office

Office européen des brevets

(11) Numéro de publication: **0 502 767 B1**

# FASCICULE DE BREVET EUROPEEN

(45) Date de publication de fascicule du brevet: **03.05.95**

(51) Int. Cl.[6]: **C07C 11/02**, C07C 1/20, B04C 5/081

(21) Numéro de dépôt: **92400521.8**

(22) Date de dépôt: **27.02.92**

(54) **Procédé et dispositif pour la conversion d'un composé oxygéné en hydrocarbures oléfiniques.**

(30) Priorité: **07.03.91 FR 9102892**

(43) Date de publication de la demande:
**09.09.92 Bulletin 92/37**

(45) Mention de la délivrance du brevet:
**03.05.95 Bulletin 95/18**

(84) Etats contractants désignés:
**DE ES GB IT NL**

(56) Documents cités:
**US-A- 4 197 418**

(73) Titulaire: **INSTITUT FRANCAIS DU PETROLE**
**4, avenue de Bois Préau**
**F-92502 Rueil-Malmaison (FR)**

(72) Inventeur: **Hugues, François**
**10 Chemin du Clos Challans,**
**Charly**
**F-69390 Vernaison (FR)**
Inventeur: **Burzynski, Jean-Pierre**
**25, Rue du Brulet**
**F-69110 Sainte Foy Les Lyon (FR)**
Inventeur: **Vuillemot, Daniel**
**27, Rue du l'Haye**
**F-69230 St Genis Laval (FR)**
Inventeur: **Galtier, Pierre**
**Centre Hospitalier Mont Salamon,**
**BP 12**
**F-38209 Vienne Cédex (FR)**
Inventeur: **Gauthier, Thierry**
**13, Allée des Muses**
**F-69230 Saint Genis Laval (FR)**

# Description

L'invention concerne un procédé de conversion catalytique, en lit entraîné, d'une charge comprenant au moins un composé oxygéné et plus spécialement au moins un alcool tel que le méthanol et/ou au moins un éther-oxyde tel que le diméthyléther, et un dispositif, pour la mise en oeuvre de ce procédé comprenant au moins un mélangeur-séparateur cyclonique à cocourant permettant la séparation très rapide des particules solides et des gaz et la trempe desdits gaz.

Ce procédé s'applique plus particulièrement à la production d'hydrocarbures oléfiniques, notamment à usage pétrochimique, comprenant une majeure partie de composés ayant de 2 à 4 atomes de carbone dans leur molécule, en présence d'un catalyseur comme une zéolithe circulant dans un réacteur à lit entraîné.

Une utilisation particulière donnée ici à titre d'exemple est la conversion catalytique du méthanol en hydrocarbures riches en éthylène et/ou en propylène.

L'art antérieur est illustré par exemple dans le brevet US-A-4 197 418.

Un des facteurs essentiels à la production sélective d'oléfines légères, et plus particulièrement d'éthylène et/ou de propylène par conversion du méthanol, est la maîtrise du temps de contact entre la charge et le catalyseur. Ce contact entre la charge et le catalyseur doit être à la fois court et uniforme (ou régulier) dans le temps, ce qui impose une rapidité et une uniformité de la mise en contact entre les grains de catalyseur et la charge gazeuse ou liquide. Il est donc important de bien contrôler, en particulier, la quantité de charge introduite en liaison avec la quantité de catalyseur avec lequel elle entre en contact, et de garder constant le temps de contact entre la charge et le catalyseur de manière à obtenir la meilleure sélectivité possible en le ou les composés éthyléniques souhaités. En l'absence d'un tel contrôle la tendance de la réaction est de conduire à une large gamme d'hydrocarbures saturés et insaturés allant généralement du méthane à des hydrocarbures ayant jusqu'à 10 atomes de carbone dans leur molécule ou plus, tels que ceux constituant habituellement la coupe essence.

Dans les procédés de conversion du méthanol utilisant la technique du lit entraîné décrits dans l'art antérieur, on insiste le plus souvent sur l'importance de l'optimisation et du contrôle du temps de contact entre les particules catalytiques et la charge. Ce temps de contact doit être, comme cela est par exemple mentionné dans le brevet US-A-4229608, relativement court. Cependant on ne mentionne pas l'utilisation d'un système de séparation et de trempe permettant une séparation efficace et rapide des particules solides et des gaz contenant les produits de la réaction et la trempe rapide desdits gaz.

L'association de conditions opératoires bien choisies, d'une séparation ultra-rapide des particules catalytiques et des produits gazeux incluant les produits de la conversion et d'une trempe rapide et efficace desdits produits gazeux permet d'éviter largement une évolution ultérieure des produits formés vers des produits de poids moléculaire plus élevé et également d'améliorer sensiblement la sélectivité en le ou les composés éthyléniques souhaités, ce qui n'était pas le cas dans les réalisations décrites dans l'art antérieur telle que par exemple celle décrite dans le brevet US-A-4046825.

De façon plus précise la présente invention concerne un procédé de conversion catalytique d'une charge comprenant au moins un composé oxygéné, en hydrocarbures oléfiniques riches en composés ayant de 2 à 4 atomes de carbone dans leur molécule comprenant la conversion en lit entraîné de ladite charge, dans une zone réactionnelle de conversion de forme allongée, dans des conditions appropriées, en présence d'un catalyseur sous forme de particules solides, ledit procédé comportant :

- a) une étape d'alimentation, dans une zone située à proximité d'une première extrémité de ladite zone réactionnelle, en au moins une phase dense D1 sous forme de particules solides contenant des particules catalytiques,
- b) une étape d'alimentation, dans une zone située à proximité d'une première extrémité de ladite zone réactionnelle, en au moins un fluide d'entraînement,
- c) une étape d'introduction, et, dans le cas d'une charge au moins en partie liquide, de pulvérisation de ladite charge dans une zone d'introduction située en aval, dans le sens du déplacement des particules solides, de la zone d'introduction desdites particules solides,
- d) une étape de mise en contact desdites particules solides et de ladite charge dans une zone située à proximité de la première extrémité,
- e) une étape de circulation des particules solides et de la charge dans la zone de conversion au cours de laquelle on effectue la conversion de ladite charge et on désactive au moins en partie les particules solides catalytiques par dépôt de coke sur celles-ci,
- f) une étape de séparation, à partir de leur mélange M1, de la phase dense D1 et des gaz, formant une phase légère L1, contenant les produits de la conversion, au moins partielle, de ladite charge, dans une zone de séparation et de trempe située à proximité d'une deuxième extrémité de la zone de conversion à l'opposé de ladite première extrémité,

- g) une étape de régénération, dans au moins une zone de régénération, d'au moins une partie des particules solides catalytiques contenues dans la phase dense D1, au moins en partie désactivées et

- h) une étape de recyclage de ladite phase dense D1 contenant les particules solides catalytiques au moins en partie régénérées, dans une zone de recyclage à proximité de ladite première extrémité,

caractérisé en ce que au cours de l'étape f) on sépare la phase dense D1 de la phase légère L1 contenant les produits de la conversion et on effectue la trempe de ladite phase légère dans une zone de séparation et de trempe comportant en combinaison :

- au moins une enceinte extérieure, de forme allongée le long d'au moins un axe, de section sensiblement circulaire de diamètre (Dc) et de longueur (L), dans laquelle on introduit à proximité d'une première extrémité de ladite enceinte extérieure, par une entrée (1) dite entrée externe, le mélange M1 que l'on fait circuler, depuis ladite première extrémité vers une deuxième extrémité opposée à ladite première extrémité de ladite enceinte extérieure, en conférant au moins à la phase L1 dudit mélange un mouvement hélicoïdal dans le sens de l'écoulement dudit mélange M1, on sépare la phase dense D1 de la phase légère L1, on récupère, par une sortie (7) dite sortie externe, au moins une partie de la phase dense D1 à proximité de ladite deuxième extrémité et on envoie la phase légère L1 dans l'entrée (4), dite deuxième entrée interne, de la deuxième enceinte intérieure décrite ci-après,

- au moins une première enceinte intérieure, de forme allongée le long d'au moins un axe, de section sensiblement circulaire, de longueur (Li) inférieure à (L), disposée coaxialement par rapport à ladite enceinte extérieure, dans laquelle on introduit, par une entrée (3) dite première entrée interne, à proximité de l'entrée externe de l'enceinte extérieure, une phase légère L2 ou une phase dense D2 ou un mélange M2 comprenant à la fois une phase légère L2 et une phase dense D2, les dites phases L2 ou D2 ou ledit mélange M2 ayant une température inférieure à la température de la phase L1, on fait circuler, dans la même direction que le mélange M1, lesdites phases L2 ou D2 ou ledit mélange M2 depuis ladite première entrée interne jusqu'à une première sortie interne (3'), de diamètre interne (Di) inférieur à (Dc), opposée à ladite première entrée interne (3), par laquelle ladite phase L2 ou ladite phase D2 ou ledit mélange M2 quitte par ladite sortie (3') ladite première enceinte intérieure,

- au moins une deuxième enceinte intérieure de forme allongée le long d'au moins un axe, de section sensiblement circulaire, disposée coaxialement par rapport à ladite première enceinte intérieure, comprenant une première extrémité située à une distance (Le) de la première sortie interne (3') de la première enceinte intérieure, ladite distance (Le) étant telle que la somme des distances (Le) et (Li) soit au plus égale à (L), dans laquelle pénètre, par une entrée (4) dite deuxième entrée interne, de diamètre interne (De) supérieur ou égal à (Di) et inférieur à (Dc), au moins une partie de la phase légère L1 et au moins une partie de la phase légère L2 ou de la phase dense D2 ou du mélange M2, et on évacue de ladite deuxième enceinte, par une deuxième sortie interne (4') opposée à ladite deuxième entrée interne, un mélange M3 comprenant au moins une partie de la phase légère L1 et au moins une partie de la phase légère L2 ou de la phase dense D2 ou du mélange M2, et

- on envoie le mélange M3 dans une zone dans laquelle on sépare et on récupère d'une part ladite phase L2 ou ladite phase D2 ou ledit mélange M2 et d'autre part la phase L1 refroidie contenant les hydrocarbures oléfiniques formés au cours de la conversion.

Dans une forme préférée de mise en oeuvre du procédé de la présente invention, la distance (Le) entre la première sortie interne (3') de la première enceinte intérieure et la première entrée interne (4) de la deuxième enceinte intérieure, est d'environ 0,1 x(Dc) à environ 10x(Dc).

Dans une forme préférée de mise en oeuvre du procédé de la présente invention le mélange M1 est introduit dans la zone de séparation et de trempe selon une direction sensiblement perpendiculaire à l'axe de son enceinte extérieure ou selon une direction sensiblement parallèle à l'axe de ladite enceinte extérieure.

Dans une forme particulière de réalisation du procédé selon la présente invention on introduit, par exemple par l'intermédiaire d'au moins un moyen d'introduction, dans la zone de séparation et de trempe au moins un fluide (L3) permettant d'effectuer simultanément le stripage des particules solides de la phase dense D1. Ce fluide est habituellement un gaz choisi parmi les gaz employés de façon classique par les hommes du métier pour effectuer un tel stripage. Ce gaz sera par exemple de la vapeur d'eau ou un gaz inerte tel que par exemple de l'azote.

Dans le procédé de la présente invention, la séparation de la phase dense D1 et la trempe de la phase légère L1 sont effectuées dans un appareil unique dénommé ci-après soit mélangeur-séparateur cyclonique à co-courant soit l'appareil.

Ce type d'appareil permet, contrairement au cas des appareils utilisés dans l'art antérieur pour effectuer la séparation et notamment au cas où l'appareil employé pour effectuer la séparation est un cyclone à rebours, en plaçant l'entrée interne de la phase légère L1 (essentiellement gazeuse) assez près de l'entrée (à une distance inférieure à la longueur (Lc) du cyclone à rebours), du mélange M1 (contenant les particules solides formant une phase dense D1 et les gaz formant une phase légère L1) et en contrôlant la circulation de la phase légère dans l'entrée interne et l'écoulement dans l'entrée du mélange M1, d'obtenir une séparation rapide des phases tout en conservant une bonne efficacité de la collecte de la phase dense D1 et en ayant une distribution de temps de séjour de la phase légère acceptable. Par ailleurs l'appareil utilisé dans le cadre du procédé de l'invention permet de limiter largement le temps pendant lequel les gaz, séparés des solides chauds, restent à une température relativement élevée. On limite donc ainsi au maximum les réactions thermiques secondaires des oléfines formées au cours de la conversion.

Outre la simplification de l'appareillage nécessaire pour effectuer la séparation et la trempe, l'appareil utilisé dans le cadre du procédé de la présente invention permet une trempe ultrarapide et très efficace des produits gazeux.

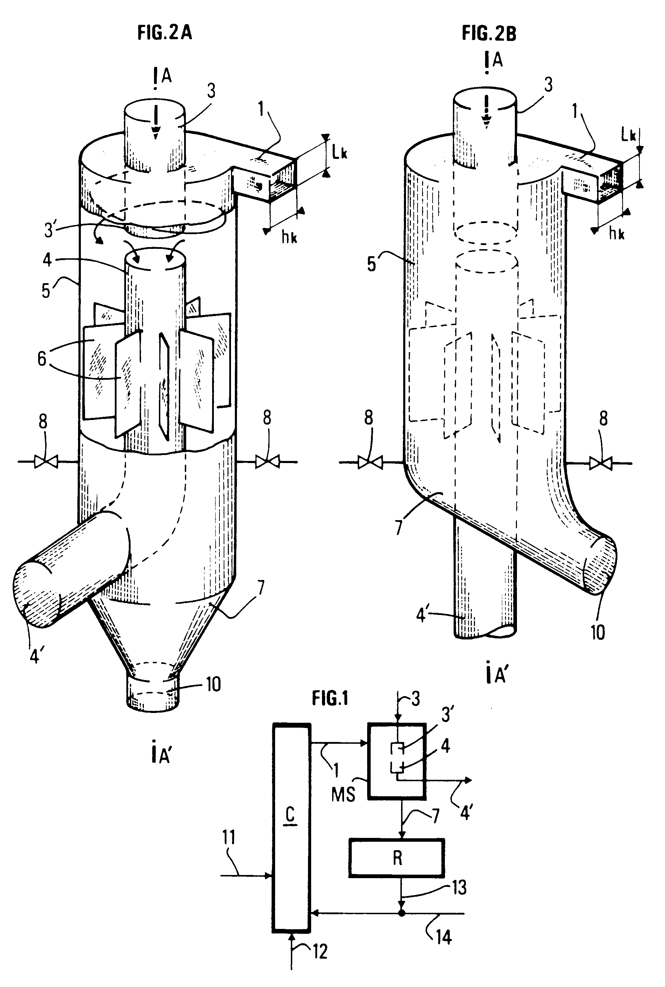

L'invention sera mieux comprise par la description de quelques modes de réalisation, donnés à titre purement illustratif mais nullement limitatif, qui en sera faite ci-après à l'aide des figures 1, 2A, 2B, 3 et 4 annexées, sur lesquelles les organes similaires sont désignés par les mêmes chiffres et lettres de référence.

La figure 1 représente schématiquement un dispositif de conversion catalytique selon l'invention dans lequel la conversion est effectuée dans un réacteur (C) dans lequel la charge est introduite par la conduite (11), les particules solides comprenant des particules solides catalytiques et formant la phase dense D1 sont introduites par le conduit (14) et le gaz d'entraînement du solide est introduit par le conduit (12) ; le réacteur est relié par un conduit (1) à un mélangeur-séparateur (MS) cyclonique à co-courant permettant de séparer une phase solide d'une phase gazeuse, ladite phase gazeuse contenant les produits de la conversion et étant récupérée par le conduit (4') tout en effectuant la trempe de cette phase gazeuse par un produit M2, sous la forme d'une phase gazeuse, liquide et/ou solide, introduit par le conduit (3) et qui circule dans une première enceinte intérieure jusqu'à la sortie (3') puis pénètre par l'entrée (4) dans une deuxième enceinte intérieure dans laquelle elle circule jusqu'à la sortie (4').

La phase dense D1 est envoyée, par le conduit (7), à un régénérateur (R) dans lequel les particules catalytiques sont au moins en partie régénérées avant d'être renvoyées, par le conduit (13) relié au conduit (14), dans le réacteur (C). Le mélange de la phase gazeuse contenant les produits de la conversion et du produit M2 est envoyé à un séparateur dans lequel on sépare le produit M2 de ladite phase gazeuse, et on recycle, éventuellement après refroidissement, le produit M2 au mélangeur-séparateur (MS).

La figure 2A est une vue en perspective d'un mélangeur-séparateur cyclonique à co-courant utilisé dans une forme préférée selon l'invention.

La figure 2B est une vue en perspective d'un appareil utilisé dans le cadre de la présente invention qui ne diffère de celui représenté sur la figure 2A que par les moyens de sortie (7) de la phase dense D1 introduite par le conduit (1), lesdits moyens (7) permettant dans le mode de réalisation schématisé sur la figure 2B une sortie latérale (10) de la phase dense D1 et dans celui schématisé sur la figure 2A une sortie axiale (10) de cette phase.

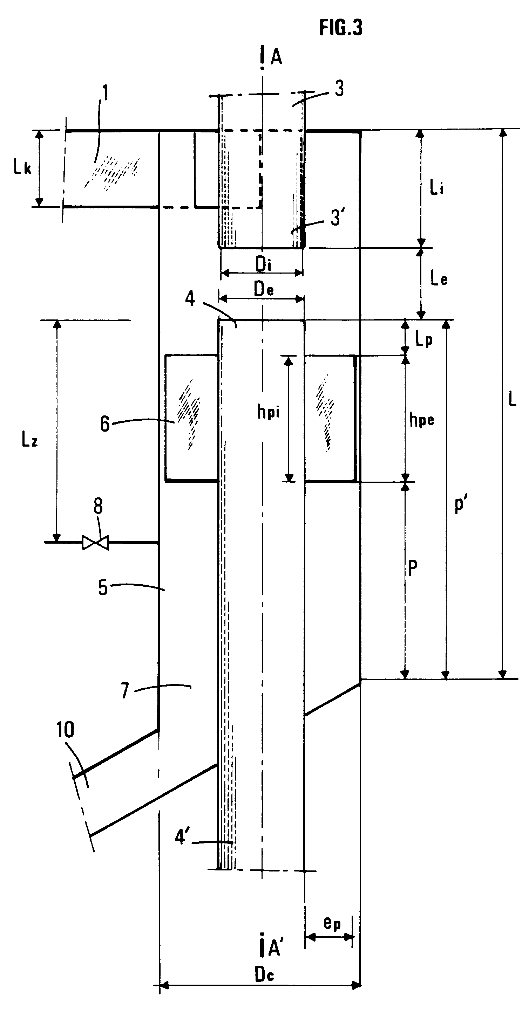

La figure 3 est une vue en coupe d'un appareil, utilisé dans le procédé selon l'invention, pratiquement identique à celui représenté sur la figure 2B, comportant des moyens (6) limitant la progression de la phase légère L1 à l'extérieur de l'enceinte intérieure. Dans le cas schématisé sur la figure 3 la dimension desdits moyens (6), dans la direction perpendiculaire à l'axe de l'enceinte extérieure, est inférieure à la dimension de la sortie externe (5).

Les appareils, utilisés dans le cadre de l'invention, schématisés sur les figures 2A et 3, de forme allongée, sensiblement régulière, le long d'un axe (AA') qui est un axe de symétrie, comportent une enceinte extérieure, de diamètre (Dc) et de longueur (L) ayant une entrée tangentielle (1) dite entrée externe, dans laquelle on introduit, suivant une direction sensiblement perpendiculaire à l'axe de l'appareil, le mélange M1 contenant au moins une phase dense D1 et au moins une phase légère L1. Cette entrée tangentielle a de préférence une section rectangulaire ou carrée dont le côté parallèle à l'axe de l'appareil a une dimension (Lk) habituellement d'environ 0,25 à environ 1 fois le diamètre (Dc), et le côté perpendiculaire à l'axe de l'appareil a une dimension (hk) habituellement d'environ 0,05 à environ 0,5 fois le diamètre (Dc).

Le mélange M1 ainsi introduit s'enroule autour d'une première enceinte intérieure, disposée coaxialement par rapport à l'enceinte extérieure, ayant une entrée axiale (3), dite première entrée interne, permettant l'introduction d'une phase légère L2 ou d'une phase dense D2 ou de préférence d'un mélange M2 contenant une phase dense D2 et une phase légère L2. Cette phase légère L2 ou cette phase dense D2 ou ce mélange M2 circule parallèlement à l'axe (AA') de l'appareil jusqu'à la première sortie interne (3') de diamètre interne (Di) inférieur au diamètre (Dc) de l'enceinte extérieure de l'appareil et habituellement d'environ 0,05 à environ 0,9 fois ce diamètre (Dc) et de préférence d'environ 0,4 à environ 0,8 fois ce diamètre (Dc).

La longueur (Li), entre le niveau extrême de l'entrée tangentielle (1) et la première sortie interne, est inférieure à (L) et est habituellement d'environ 0,2 à environ 9,5 fois le diamètre (Dc) et de préférence d'environ 1 à environ 3 fois ce diamètre (Dc).

Bien que cela ne soit pas réprésenté sur les figures 2A, 2B et 3 il est possible, et habituellement souhaitable, dans le cas de débits importants des diverses phases au niveau des entrées de l'appareil, d'utiliser des moyens permettant de favoriser la formation du vortex comme par exemple un toit hélicoïdal descendant à partir du niveau extrême de l'entrée tangentielle (1) ou une volute par exemple externe, et permettant de limiter la turbulence au niveau de l'entrée tangentielle (1). Habituellement le pas de l'hélice est d'environ 0,01 à environ 3 fois la valeur de (Lk) et le plus souvent d'environ 0,5 à environ 1,5 fois cette valeur.

La phase légère L2 ou la phase dense D2 ou le mélange M2 pénétre ensuite au moins en partie dans la deuxième enceinte intérieure, disposée coaxialement par rapport à la première enceinte intérieure, par la deuxième entrée interne (4) située à une distance (Le) de la première sortie interne (3'), cette distance étant de préférence d'environ 0,2 à environ 2 fois le diamètre (Dc). Dans cette deuxième enceinte pénétre également au moins une partie de la phase légère L1. Cette deuxième entrée interne (4) a un diamètre interne (De) supérieur ou égal à (Di) et inférieur à (Dc) et habituellement d'environ 0,2 à environ 0,9 fois le diamètre (Dc). Ce diamètre (De) est de préférence d'environ 0,4 à environ 0,8 fois le diamètre (Dc). On récupère par la deuxième sortie interne (4') de l'appareil un mélange comprenant au moins une partie de la phase légère L1 et au moins une partie de la phase légère L2 ou de la phase dense D2 ou du mélange M2 comprenant une phase dense D2 et une phase légère L2.

Selon la réalisation schématisée sur les figures 2A et 3 l'appareil comporte, en aval, dans le sens de l'écoulement des diverses phases, de la deuxième entrée interne, au moins un moyen (6) limitant la progression de la phase légère L1 dans l'espace situé entre la paroi interne de l'enceinte extérieure et la paroi externe de la deuxième enceinte intérieure ou sortie externe (5). Ce ou ces moyens (6) sont de préférence des pales sensiblement planes dont le plan comprend l'axe de l'appareil. Ces moyens (6) sont habituellement fixés sur au moins une paroi de l'une des enceintes intérieure ou extérieure. Ces moyens (6) sont de préférence fixés à la paroi externe de la deuxième enceinte intérieure de sorte que la distance (Lp) entre la deuxième entrée interne et le point desdites pales le plus proche de cette deuxième entrée interne soit d'environ 0 à environ 5 fois le diamètre (Dc) et de préférence d'environ 0,1 à environ 1 fois ce diamètre (Dc).

Le nombre de pales est variable suivant la distribution du temps de séjour que l'on accepte pour la phase L1 et également en fonction du diamètre (Dc) de l'enceinte extérieure. Si le temps de séjour de la phase L1 peut avoir une distribution large il ne sera alors pas indispensable d'avoir des pales. Le nombre de pales est habituellement compris entre 0 et environ 50, le plus souvent, lorsque des pales sont présentes, d'au moins 2 et par exemple de 2 à environ 50 et de préférence de 3 à environ 50. Les pales permettent par une limitation de la continuation du vortex sur toute la section du cyclone, autour de la sortie interne (4) de la phase légère, une diminution et un contrôle de la distribution des temps de séjour et, par voie de conséquence, on limitera la dégradation des produits contenus dans la phase légère circulant autour de la sortie interne.

Chacune de ces pales a habituellement une dimension ou largeur (ep) mesurée dans la direction perpendiculaire à l'axe de l'appareil et définie par rapport au diamètre intérieur (Dc) de l'enceinte extérieure et au diamètre extérieur (De) de la deuxième enceinte intérieure, d'environ 0,01 à 1 fois la valeur $[((Dc)-(De))/2)]$ de la demi différence de ces diamètres (Dc) et (De), de préférence d'environ 0,5 à 1 fois cette valeur et le plus souvent d'environ 0,9 à 1 fois cette valeur.

Dans le cas d'un appareil vertical, utilisé dans le procédé selon l'invention, tel que par exemple celui schématisé sur la figure 2A, ayant une sortie interne (4') latérale, et lorsque les pales sont positionnées après cette sortie interne, cette dimension (ep) peut être d'environ 0,01 à environ 1 fois la valeur (Dc)/2 du demi diamètre de l'enceinte extérieure.

Ces pales ont chacune sur leur arête, la plus proche de l'axe des enceintes intérieures, dans la direction parallèle à cet axe, une dimension ou hauteur interne (hpi) mesurée dans la direction de l'axe de l'appareil sur l'arête de ladite pale la plus proche de l'axe des enceintes intérieures et une dimension ou hauteur externe (hpe) mesurée dans la direction de l'axe de l'appareil sur l'arête de ladite pale la plus proche de la paroi interne de l'enceinte extérieure. Ces dimensions (hpi) et (hpe) sont habituellement supérieures à 0,1 fois le diamètre (Dc) et par exemple d'environ 0,1 fois à environ 10 fois le diamètre (Dc) et le plus souvent d'environ 1 à environ 4 fois ce diamètre (Dc). De préférence ces pales ont chacune une dimension (hpi) supérieure ou égale à leur dimension (hpe).

Selon la réalisation schématisée sur les figures 2A et 3 l'appareil comporte, en aval, dans le sens de l'écoulement des diverses phases, de la deuxième entrée interne, au moins un moyen (8) permettant l'introduction éventuelle d'une phase légère L3 en au moins un point situé entre la deuxième entrée interne (4) de la deuxième enceinte intérieure et la sortie externe (10) de la phase dense D1 ; ce ou ces points sont de préférence à une distance (Lz) de l'entrée (4) de la deuxième enceinte intérieure. Ladite distance (Lz) a de préférence une valeur au moins égale à la somme des valeurs de (Lp) et (hpi) et au plus égale à la distance entre l'entrée (4) de la deuxième enceinte intérieure et les moyens de sortie (7) de la phase dense D1. Cette phase légère L3 peut être introduite par exemple dans le cas où il est souhaitable d'effectuer un stripage de la phase dense D1. La phase légère L3 est de préférence introduite en plusieurs points qui sont habituellement répartis symétriquement, dans un plan au niveau duquel l'introduction est effectuée, autour de l'enceinte extérieure.

Le ou les points d'introduction de cette phase légère L3 sont habituellement situés à une distance au moins égale à 0,1 fois le diamètre (Dc) de l'entrée (4) de la deuxième enceinte intérieure lorsque l'appareil ne comporte pas de moyens (6) ou du point desdits moyens (6) le plus proche des moyens (7) de sortie de la phase dense D1 lorsque l'appareil comporte des moyens (6). Le ou les points d'introduction de cette phase légère L3 sont de préférence situé à proximité de la sortie externe (10) et le plus souvent à proximité des moyens de sortie (7) de la phase dense D1.

La dimension (p') entre le niveau de la deuxième entrée interne (4) et les moyens (7) de sortie de la phase dense D1 est déterminée à partir des autres dimensions des divers moyens formant l'appareil et de la longueur (L) de l'enceinte extérieure mesurée entre le niveau extrême de l'entrée tangentielle (1) et les moyens (7) de sortie de la phase dense D1. Cette dimension (L) est habituellement d'environ 1 à environ 35 fois le diamètre (Dc) de l'enceinte extérieure et le plus souvent d'environ 1 à 25 fois ce diamètre (Dc). On peut de même calculer la dimension (P), entre le point des moyens (6) le plus proche des moyens (7) de sortie de la phase dense D1 et lesdits moyens (7), à partir des autres dimensions des divers moyens formant l'appareil et de la longueur (L).

On ne sortirait pas du cadre de la présente invention dans le cas où l'axe (AA') de l'appareil fait un angle avec la verticale. Dans ce cas il est cependant préférable, si des moyens (6), limitant la circulation de la phase légère L1 dans la sortie externe (5) et donc réduisant la distribution des temps de séjour de cette phase L1 dans l'appareil, sont utilisés, de les placer verticalement et donc de réaliser un appareil comportant, dans le cas d'une sortie interne (4') axiale, un coude au delà duquel lesdits moyens (6) seront positionnés dans la sortie externe verticale. De même dans le cas d'un appareil tel que celui schématisé sur la figure 2A ayant une sortie latérale (4'), il est possible de positionner les moyens (6), limitant la circulation de la phase légère L1 dans la sortie externe (5) et donc réduisant la distribution des temps de séjour de cette phase L1 dans l'appareil, après le niveau de la sortie interne (4') et avant les moyens (7).

Les moyens (6) limitent la progression du vortex de la phase légère L1 dans la sortie externe (5). La position de ces moyens (6) et leur nombre influent donc sur les performances de la séparation des phases D1 et L1 contenues dans le mélange M1 (perte de charge et efficacité de la collecte de la phase dense D1) et également sur la pénétration du vortex de la phase légère L1 dans la sortie (5). Ces paramètres seront donc choisis avec soin par l'homme du métier en particulier en fonction des résultats souhaités et de la perte de charge tolérée. En particulier le nombre de pales, leur forme et leur position seront choisis avec soins en tenant compte de leur influence sur l'écoulement du solide D1 en liaison avec la limitation recherchée de la progression du vortex dans la sortie externe (5).

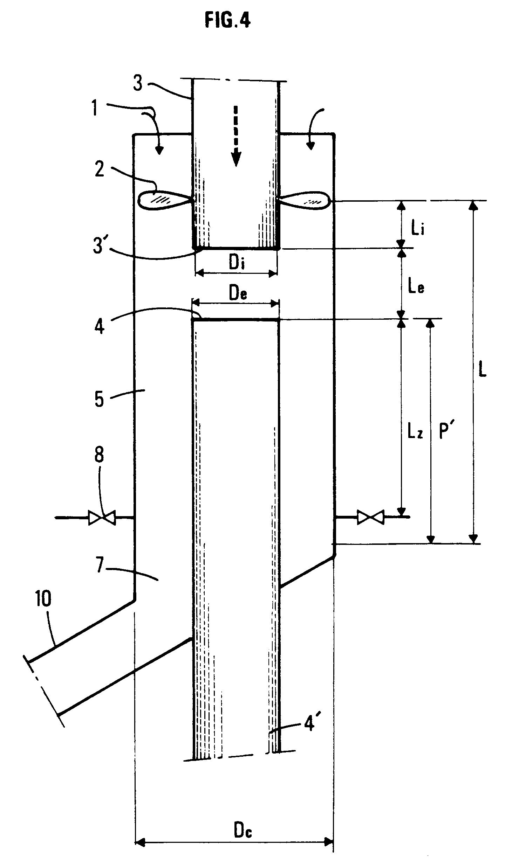

La figure 4 est une vue en perspective d'un appareil selon l'invention comportant une enceinte extérieure, de diamètre (Dc) ayant une entrée (1) dite entrée externe axiale, dans laquelle on introduit suivant une direction sensiblement parallèle à l'axe (AA') de l'appareil le mélange M1 contenant une phase dense D1 et une phase légère L1. Cet appareil comporte en outre des moyens (2) placés à l'intérieur de l'entrée (1) dans l'espace situé entre la paroi interne de l'enceinte extérieure et la paroi externe de la première enceinte intérieure permettant de conférer en aval, dans le sens de circulation dudit mélange M1, un mouvement hélicoïdal ou tourbillonnant au moins à la phase L1 dudit mélange M1. Ces moyens sont habituellement des pales inclinées. La longueur L de l'appareil est comptée entre ces moyens permettant de créer un vortex, au moins sur la phase L1, et les moyens (7) de sortie de la phase dense D1. Cet appareil ne comporte pas de moyens (6) de limitation de la pénétration du vortex dans la sortie externe (5). Toutes les autres caractéristiques sont identiques à celles décrites en liaison avec les appareils représentés sur les figures 2A et 3, en particulier les diverses dimensions sont celles mentionnées dans la description de ces appareils. Les variantes décrites en liaison avec les appareils représentés sur les figures 2A et 3 sont également possibles dans le cas de l'appareil selon la présente invention schématisé sur la figure 4. On peut en particulier envisager une sortie interne (4') latérale et une sortie externe axiale (10) comme dans le cas de la réalisation schématisée sur la figure 2A, et également l'utilisation de moyens (6) dans la sortie externe (5).

Les moyens de sortie (7) de la phase dense D1 permettent habituellement de collecter et de canaliser cette phase dense D1 jusqu'à la sortie externe (10). Ces moyens sont le plus souvent un fond incliné ou un cône axé ou non sur la sortie interne (4').

Les appareils de la présente invention schématisés sur les figures annexées comportent un axe unique (AA') mais on ne sortirait pas du cadre de la présente invention dans le cas où l'on réaliserait un appareil comportant plusieurs axes par exemple faisant un angle entre eux. Dans ce cas l'axe (AA') mentionné ci-avant serait l'axe de la partie de l'appareil située entre la première entrée interne (3) et le première sortie interne (3') et la valeur du diamètre (Dc) serait celle mesurée au niveau de cette sortie interne (3'), cet axe (AA') restant aussi dans ce cas l'axe de la deuxième enceinte intérieure, les deux enceintes intérieures restant disposées coaxialement (un tel cas est par exemple celui d'un appareil comportant une enceinte extérieure coudée).

Le diamètre (Dc) de l'appareil mesuré au niveau de la première sortie interne (3') est habituellement d'environ 0,01 à environ 10 m (mètres) et le plus souvent d'environ 0,05 à environ 2 m. Il est habituellement préférable de garder un diamètre constant sur toute la longueur (L) de l'appareil ou même depuis le niveau de l'injection du mélange M1 jusqu'au niveau des moyens (7) de sortie de la phase dense D1 ; cependant on ne sortirait pas du cadre de l'invention dans le cas d'un appareil comportant des élargissements ou des rétrécissements de section entre lesdits niveaux.

Pour obtenir une bonne séparation de la phase L1 contenue dans le mélange M1 comprenant également la phase D1 et un mélange efficace de cette phase L1 avec une phase L2 ou avec une phase D2 ou avec un mélange M2 de ces phases L2 et D2 il est préférable d'avoir une vitesse superficielle d'entrée de cette phase L1 élevée. Cette vitesse sera par exemple d'environ 0,1 à environ 250 mxs$^{-1}$ (mètre par seconde) et de préférence d'environ 0,5 à environ 75 mxs$^{-1}$ et le plus souvent d'environ 1 à environ 20 mxs$^{-1}$. Le rapport en poids du débit de la phase D1 au débit de la phase L1 est habituellement d'environ 2 : 1 à environ 100 : 1 et le plus souvent d'environ 5 : 1 à environ 50 : 1. Le débit de la phase L2 ou de la phase D2 ou du mélange M2 représente habituellement en poids d'environ 0,1 à environ 1000 % du débit de la phase D1 et le plus souvent d'environ 10 à environ 300% du débit de la phase D1. La vitesse superficielle V2 de la phase L2, lorsqu'elle est présente seule ou au sein du mélange M2, est habituellement d'environ 1 à environ 500 % de la vitesse axiale moyenne V1 sur toute la section de diamètre (Dc) située entre la première sortie interne (3') et la deuxième entrée interne (4) définie par la relation :

$$V1 = L1/(\pi \times Dc^2/4)$$

dans laquelle L1 est exprimé en m$^3$xs$^{-1}$ (mètre cube par seconde) et Dc en m. La vitesse superficielle V2 sera de préférence d'environ 5 à environ 150 % de la vitesse V1.

Il est possible, par exemple en augmentant la pression en aval, dans le sens de la circulation de la phase légère L2 ou de la phase dense D2 ou du mélange M2, de la deuxième entrée interne (4) ou en diminuant la pression en aval, dans le sens de la circulation de la phase dense D1, des moyens (7) de sortie de cette phase, de soutirer une partie plus ou moins importante de la phase L1 avec la phase D1 et simultanément d'obtenir au niveau de la deuxième sortie (4') un mélange pratiquement complètement exempt de phase D1. Le plus souvent on soutire environ 1 à environ 10 % de cette phase L1 avec la phase D1. Les variations de pression permettant de jouer sur la quantité de phase L1 soutirée avec la phase D1 sont assurées par des moyens bien connus de l'homme du métier et par exemple en jouant sur la température de la trempe par modification des débits de phases L2 ou D2 ou du mélange M2, ou en modifiant le débit de la phase L3, ou en modifiant les conditions d'opération en aval de la sortie (10).

Dans les divers appareils utilisés dans le cadre de la présente invention et dans les différents modes d'injection du mélange M1, un tel soutirage peut permettre d'améliorer l'efficacité de récupération de la phase dense D1. Ainsi dans une forme avantageuse de réalisation de l'invention l'appareil comprendra au moins un moyen permettant le soutirage, par la sortie externe, d'au moins une partie de la phase légère L1 en mélange avec la phase dense D1.

Le choix entre un appareil comportant une entrée tangentielle, pour le mélange M1, et un appareil comportant une entrée axiale, pour ce mélange M1, est habituellement guidé par le rapport en poids des débits des phases L1 et D1. Dans le cas où ce rapport est inférieur à 5 : 1, il peut être avantageux de choisir un appareil à entrée axiale.

La présente invention concerne également un dispositif pour la conversion catalytique en lit entraîné d'une charge contenant au moins un composé oxygéné, en hydrocarbures oléfiniques riches en composés ayant de 2 à 4 atomes de carbone dans leur molécule. comportant une enceinte de forme allongée, dans laquelle ladite conversion est effectuée dans des conditions appropriées, comprenant, à proximité d'une première extrémité, d'amont en aval dans le sens du déplacement de la charge, au moins un moyen d'introduction d'au moins un fluide de d'entraînement, au moins un moyen d'introduction d'au moins un solide formant une phase dense D1 contenant des particules catalytiques, au moins un moyen d'introduction de ladite charge, ledit dispositif comprenant à proximité d'une deuxième extrémité de ladite enceinte au moins un moyen, relié à ladite enceinte, de séparation à partir de leur mélange M1 des particules solides et des gaz formant une phase légère L1 et de trempe desdits gaz contenant les produits de la conversion, au moins partielle, de ladite charge et au moins un moyen de régénération d'au moins une partie des particules solides catalytiques relié d'une part audit moyen de séparation et de trempe et d'autre part à ladite enceinte à proximité de ladite première extrémité de manière à permettre la régénération d'au moins une partie des particules solides catalytiques et le recyclage desdites particules solides dans ladite enceinte caractérisé en ce que ledit moyen de séparation des particules solides et de trempe des gaz est un mélangeur-séparateur cyclonique à co-courant comportant en combinaison :

- au moins une enceinte extérieure, de forme allongée le long d'au moins un axe, de section sensiblement circulaire de diamètre (Dc) et de longueur (L), comportant à proximité d'une première extrémité au moins un moyen d'introduction permettant d'introduire, par une entrée (1) dite entrée externe, le mélange M1 contenant la phase dense D1 et la phase légère L1, ledit moyen étant adapté à conférer au moins à la phase L1 un mouvement hélicoïdal dans le sens de l'écoulement dudit mélange M1 dans ladite enceinte extérieure et comprenant également des moyens de séparation des phases D1 et L1, et à proximité d'une deuxième extrémité opposée à ladite première extrémité des moyens de récupération permettant de récupérer, par une sortie (7) dite sortie externe, au moins une partie de la phase dense D1,

- au moins une première enceinte intérieure, de forme allongée le long d'au moins un axe, de section sensiblement circulaire, de longueur (Li) inférieure à (L), disposée coaxialement par rapport à ladite enceinte extérieure, comprenant à une entrée (3), dite première entrée interne, située à proximité de l'entrée externe de l'enceinte extérieure, au moins un moyen d'introduction permettant d'introduire par ladite première entrée interne une phase légère L2 ou une phase dense D2 ou un mélange M2 comprenant à la fois une phase légère L2 et une phase dense D2, lesdites phases L2 ou D2 ou ledit mélange M2 ayant une température inférieure à la température de la phase L1, lesdits moyens permettant d'introduire ladite phase L2 ou D2 ou ledit mélange M2 de façon à ce que leur écoulement ait lieu dans la même direction que l'écoulement du mélange M1 jusqu'à une première sortie interne (3'), de diamètre interne (Di) inférieur à (Dc), opposée à ladite première entrée interne (3), par laquelle ladite phase L2 ou ladite phase D2 ou ledit mélange M2 quitte par ladite sortie (3') ladite première enceinte intérieure,

- au moins une deuxième enceinte intérieure de forme allongée le long d'au moins un axe, de section sensiblement circulaire, disposée coaxialement par rapport à ladite première enceinte intérieure, comprenant une première extrémité située à une distance (Le) de la première sortie interne (3') de la première enceinte intérieure, ladite distance (Le) étant telle que la somme des distances (Le) et (Li) soit au plus égale à (L), dans laquelle pénètre, par une entrée (4) dite deuxième entrée interne, de diamètre interne (De) supérieur ou égal à (Di) et inférieur à (Dc), au moins une partie de la phase légère L1 et au moins une partie de la phase légère L2 ou de la phase dense D2 ou du mélange M2, ladite deuxième enceinte intérieure comprenant à une sortie (4') dite deuxième sortie interne située à proximité de la sortie externe (7) de l'enceinte extérieure, au moins un moyen d'évacuation permettant d'évacuer par ladite deuxième sortie interne, un mélange M3 comprenant au moins une partie de la phase légère L1 et au moins une partie de la phase légère L2 ou de la phase dense D2 ou du mélange M2, et

- au moins un moyen permettant de séparer et de récupérer à partir du mélange M3 d'une part ladite phase L2 ou ladite phase D2 ou ledit mélange M2 et d'autre part la phase L1 refroidie contenant les hydrocarbures oléfiniques formés au cours de la conversion.

Dans le cadre de la présente invention il n'est pas fait mention des solides et catalyseurs employés, ni des divers fluides utilisés qui sont des données classiques bien connues de l'homme de la conversion catalytique des composés oxygénés en hydrocarbures oléfiniques. Le fluide d'entraînement est le plus souvent choisi dans le groupe formé par la vapeur d'eau, les gaz inertes ou les hydrocarbures gazeux ou des mélanges de ces composés. Les conditions de mise en oeuvre de la réaction sont souvent telles que l'on opère en lit fluidisé entraîné. Dans ce cas le fluide de fluidisation est le plus souvent le même que le fluide d'entraînement. Ce fluide unique est alors divisé en deux fractions l'une formant le fluide de fluidisation et l'autre le fluide d'entraînement.

Les catalyseurs employés sont de préférence des catalyseurs zéolithiques.

Les conditions opératoires relatives aux procédés de conversion de charges oxygénées comme le méthanol sont par exemples décrites dans les brevets suivants : US-A-4689205, US-A-3969426 et US-A-4229608, mais aucun de ces brevets ne décrit l'utilisation d'un séparateur cyclonique à co-courant pour améliorer la rapidité de la séparation solide-gaz.

Les conditions opératoires de la conversion de la charge oxygénée sont en général les suivantes :

- la charge, qui le plus souvent contient un alcool et habituellement le méthanol, renferme généralement de 50 à 100 %, de préférence de 70 à 90 % en poids de composés oxygénés et de 0 à 50 %, de préférence de 5 à 30 % en poids d'eau. La charge peut également contenir un gaz inerte tel que par exemple l'azote seul ou en mélange avec la vapeur d'eau dans la proportion indiquée ci-avant pour la vapeur d'eau. Dans le cas le plus fréquent où la charge contient du méthanol elle peut être constituée par du méthanol brut tel que par exemple du méthanol provenant d'une unité de synthèse de ce composé à partir d'un gaz contenant du monoxyde de carbone,
- la température d'injection est habituellement égale à la température de rosée de la charge,
- la pression absolue régnant dans l'unité de conversion est habituellement de 0,1 à 0,5 mégapascal (MPa) et le plus souvent d'environ 0,2 MPa,
- la température de la zone réactionnelle est habituellement de 500 à 620 °C et le plus souvent de 550 à 590°C,
- le temps de séjour dans la zone réactionnelle est habituellement de 0,05 à 10 secondes et le plus souvent de 0,5 à 3 secondes,
- la vitesse des gaz dans la zone réactionnelle est habituellement de 0,5 à 40 m/s et le plus souvent de 1 à 20 m/s,
- la vitesse des solides dans la zone réactionnelle est habituellement voisine de celle des gaz et généralement de 0,5 à 40 m/s et le plus souvent de 1 à 20 m/s.

Dans une forme préférée de réalisation de l'invention la charge comprend au moins un alcool, de préférence le méthanol et elle est introduite à l'état gazeux.

Les conditions opératoires sont choisies pour que la composition des produits obtenus exprimée par le rendement en carbone en composés éthyléniques ayant de 2 à 4 atomes de carbone dans leur molécule soit supérieur à 70 % et de préférence supérieur à 80 %. Les produits obtenus peuvent être particulièrement riches en éthylène (rendement en carbone pour l'éthylène supérieur à 40 %) ou particulièrement riches en propylène (rendement en carbone pour le propylène supérieur à 60 %).

La masse volumique des solides dans le réacteur est habituellemnt de 10 à 700 kilogrammes par mètre cube (kg/m$^3$) et le plus souvent de 20 à 400 kg/m$^3$.

Le rapport C/O du débit massique de catalyseur au débit massique de charge est habituellement d'environ 2 : 1 à environ 50 : 1 et le plus souvent d'environ 5 : 1 à environ 30 : 1.

**Revendications**

1. Procédé de conversion catalytique d'une charge comprenant au moins un composé oxygéné, en hydrocarbures oléfiniques riches en composés ayant de 2 à 4 atomes de carbone dans leur molécule comprenant la conversion en lit entraîné de ladite charge, dans une zone réactionnelle de conversion de forme allongée, dans des conditions appropriées, en présence d'un catalyseur sous forme de particules solides, ledit procédé comportant :
   - a) une étape d'alimentation, dans une zone située à proximité d'une première extrémité de ladite zone réactionnelle, en au moins une phase dense D1 sous forme de particules solides contenant des particules catalytiques
   - b) une étape d'alimentation, dans une zone située à proximité d'une première extrémité de ladite zone réactionnelle, en au moins un fluide d'entraînement,
   - c) une étape d'introduction, et, dans le cas d'une charge au moins en partie liquide, de pulvérisation de ladite charge dans une zone d'introduction située en aval, dans le sens du déplacement des particules solides, de la zone d'introduction desdites particules solides,
   - d) une étape de mise en contact desdites particules solides et de ladite charge dans une zone située à proximité de la première extrémité,

- e) une étape de circulation des particules solides et de la charge dans la zone de conversion au cours de laquelle on effectue la conversion de ladite charge et on désactive au moins en partie les particules solides catalytiques par dépôt de coke sur celles-ci,
- f) une étape de séparation, à partir de leur mélange M1, de la phase dense D1 et des gaz, formant une phase légère L1, contenant les produits de la conversion, au moins partielle, de ladite charge, dans une zone de séparation et de trempe située à proximité d'une deuxième extrémité de la zone de conversion à l'opposé de ladite première extrémité,
- g) une étape de régénération, dans au moins une zone de régénération, d'au moins une partie des particules solides catalytiques contenus dans la phase dense D1, au moins en partie désactivées et
- h) une étape de recyclage de ladite phase dense D1 contenant les particules solides catalytiques au moins en partie régénérées, dans une zone de recyclage à proximité de ladite première extrémité

caractérisé en ce que au cours de l'étape f) on sépare la phase dense D1 de la phase légère L1 contenant les produits de la conversion et on effectue la trempe de ladite phase légère dans une zone de séparation et de trempe comportant en combinaison :

- au moins une enceinte extérieure, de forme allongée le long d'au moins un axe, de section sensiblement circulaire de diamètre (Dc) et de longueur (L), dans laquelle on introduit à proximité d'une première extrémité de ladite enceinte extérieure, par une entrée (1) dite entrée externe, le mélange M1 que l'on fait circuler, depuis ladite première extrémité vers une deuxième extrémité opposée à ladite première extrémité de ladite enceinte extérieure, en conférant au moins à la phase L1 dudit mélange un mouvement hélicoïdal dans le sens de l'écoulement dudit mélange M1, on sépare la phase dense D1 de la phase légère L1, on récupère, par une sortie (7) dite sortie externe, au moins une partie de la phase dense D1 à proximité de ladite deuxième extrémité et on envoie la phase légère L1 dans l'entrée (4), dite deuxième entrée interne, de la deuxième enceinte intérieure décrite ci-après,
- au moins une première enceinte intérieure, de forme allongée le long d'au moins un axe, de section sensiblement circulaire, de longueur (Li) inférieure à (L), disposée coaxialement par rapport à ladite enceinte extérieure, dans laquelle on introduit, par une entrée (3) dite première entrée interne, à proximité de l'entrée externe de l'enceinte extérieure, une phase légère L2 ou une phase dense D2 ou un mélange M2 comprenant à la fois une phase légère L2 et une phase dense D2, lesdites phases L2 ou D2 ou ledit mélange M2 ayant une température inférieure à la température de la phase L1, on fait circuler, dans la même direction que le mélange M1, lesdites phases L2 ou D2 ou ledit mélange M2 depuis ladite première entrée interne jusqu'à une première sortie interne (3'), de diamètre interne (Di) inférieur à (Dc), opposée à ladite première entrée interne (3), par laquelle ladite phase L2 ou ladite phase D2 ou ledit mélange M2 quitte par ladite sortie (3') ladite première enceinte intérieure,
- au moins une deuxième enceinte intérieure de forme allongée le long d'au moins un axe, de section sensiblement circulaire, disposée coaxialement par rapport à ladite première enceinte intérieure, comprenant une première extrémité située à une distance (Le) de la première sortie interne (3') de la première enceinte intérieure, ladite distance (Le) étant telle que la somme des distances (Le) et (Li) soit au plus égale à (L), dans laquelle pénètre, par une entrée (4) dite deuxième entrée interne, de diamètre interne (De) supérieur ou égal à (Di) et inférieur à (Dc), au moins une partie de la phase légère L1 et au moins une partie de la phase légère L2 ou de la phase dense D2 ou du mélange M2, et on évacue de ladite deuxième enceinte, par une deuxième sortie interne (4') opposée à ladite deuxième entrée interne, un mélange M3 comprenant au moins une partie de la phase légère L1 et au moins une partie de la phase légère L2 ou de la phase dense D2 ou du mélange M2, et
- on envoie le mélange M3 dans une zone dans laquelle on sépare et on récupère d'une part ladite phase L2 ou ladite phase D2 ou ledit mélange M2 et d'autre part la phase L1 refroidie contenant les hydrocarbures oléfiniques formés au cours de la conversion.

2. Procédé selon la revendication 1 dans lequel la distance (Le), entre la première sortie interne (3') de la première enceinte intérieure et la première entrée interne (4) de la deuxième enceinte intérieure, est d'environ 0,1x(Dc) à environ 10x(Dc).

3. Procédé selon la revendication 1 ou 2 dans lequel le mélange M1 est introduit dans la zone de séparation et de trempe selon une direction sensiblement perpendiculaire à l'axe de son enceinte extérieure ou selon une direction sensiblement parallèle à l'axe de ladite enceinte extérieure.

4. Procédé selon l'une des revendications 1 à 3 dans lequel la zone de séparation et de trempe comporte, dans l'espace situé entre la paroi externe de la deuxième enceinte intérieure et la paroi interne de l'enceinte extérieure, en aval de la deuxième entrée interne dans le sens de l'écoulement des diverses phases, au moins un moyen limitant la progression de la phase légère L1.

5. Procédé selon la revendication 4 dans lequel le ou les moyens limitant la progression de la phase légère L1 dans l'espace situé entre la paroi externe de la deuxième enceinte intérieure et la paroi interne de l'enceinte extérieure sont des pales sensiblement planes dont le plan comprend l'axe de ladite enceinte extérieure.

6. Procédé selon la revendication 5 dans lequel la zone de séparation et de trempe comporte de 2 à environ 50 pales fixées à la paroi externe de la deuxième enceinte intérieure de sorte que la distance entre la deuxième entrée interne et le point desdites pales le plus proche de cette deuxième entrée interne soit d'environ 0 à environ 5x(Dc).

7. Procédé selon la revendication 5 ou 6 dans lequel les pales ont chacune une dimension (ep), mesurée dans la direction perpendiculaire à l'axe de l'enceinte extérieure de zone de séparation et de trempe, d'environ 0,01 à environ 1 fois la valeur [((Dc)-(De))/2] correspondant à la distance entre la paroi externe de la deuxième enceinte intérieure de diamètre externe (De) et la paroi interne de l'enceinte extérieure de diamètre intérieur (Dc), une dimension (hpi), mesurée sur l'arête de la pale la plus proche de l'axe des enceintes intérieures dans la direction parallèle à cet axe, et une dimension (hpe), mesurée dans la direction parallèle à l'axe de l'enceinte extérieure de ladite zone de séparation et de trempe sur l'arête de la pale la plus proche de la paroi interne de l'enceinte extérieure, lesdites dimensions (hpi) et (hpe) étant d'environ 0,1x-(Dc) à environ 10x(Dc).

8. Procédé selon la revendication 7 dans lequel les pales ont chacune une dimension (hpi) supérieure ou égale à (hpe).

9. Procédé selon l'une des revendications 1 ou 8 dans lequel on introduit dans la zone de séparation et de trempe au moins un fluide (L3) permettant d'effectuer le stripage des particules solides de la phase dense D1.

10. Procédé selon l'une des revendications 1 à 9 dans lequel la charge comprend au moins un alcool, de préférence le méthanol et dans lequel elle est introduite à l'état gazeux.

11. Dispositif de conversion catalytique en lit entraîné d'une charge contenant au moins un composé oxygéné, en hydrocarbures oléfiniques riches en composés ayant de 2 à 4 atomes de carbone dans leur molécule, comportant une enceinte de forme allongée, dans laquelle ladite conversion est effectuée dans des conditions appropriées, comprenant, à proximité d'une première extrémité, d'amont en aval dans le sens du déplacement de la charge, au moins un moyen d'introduction d'au moins un fluide d'entraînement, au moins un moyen d'introduction d'au moins un solide formant une phase dense D1 contenant des particules catalytiques, au moins un moyen d'introduction de ladite charge, ledit dispositif comprenant à proximité d'une deuxième extrémité de ladite enceinte au moins un moyen, relié à ladite enceinte, de séparation à partir de leur mélange M1 des particules solides et des gaz formant une phase légère L1 et de trempe desdits gaz contenant les produits de la conversion, au moins partielle, de ladite charge et au moins un moyen de régénération d'au moins une partie des particules solides catalytiques relié d'une part audit moyen de séparation et de trempe et d'autre part à ladite enceinte à proximité de ladite première extrémité de manière à permettre la régénération d'au moins une partie des particules solides catalytiques et le recyclage desdites particules solides dans ladite enceinte caractérisé en ce que ledit moyen de séparation des particules solides et de trempe des gaz est un mélangeur-séparateur cyclonique à co-courant, comportant en combinaison :

- au moins une enceinte extérieure, de forme allongée le long d'au moins un axe, de section sensiblement circulaire de diamètre (Dc) et de longueur (L), comportant à proximité d'une première extrémité au moins un moyen d'introduction permettant d'introduire, par une entrée (1) dite entrée externe, le mélange M1 contenant la phase dense D1 et la phase légère L1, ledit moyen étant adapté à conférer au moins à la phase L1 un mouvement hélicoïdal dans le sens de l'écoulement dudit mélange M1 dans ladite enceinte extérieure et comprenant également des moyens de séparation des phases D1 et L1, et à proximité d'une deuxième extrémité opposée à ladite première extrémité des moyens de récupération permettant de récupérer, par une sortie (7) dite sortie externe, au moins une partie de la phase dense D1,

- au moins une première enceinte intérieure, de forme allongée le long d'au moins un axe, de section sensiblement circulaire, de longueur (Li) inférieure à (L), disposée coaxialement par rapport à ladite enceinte extérieure, comprenant à une entrée (3), dite première entrée interne, située à proximité de l'entrée externe de l'enceinte extérieure, au moins un moyen d'introduction permettant d'introduire par ladite première entrée interne une phase légère L2 ou une phase dense D2 ou un mélange M2 comprenant à la fois une phase légère L2 et une phase dense D2, lesdites phases L2 ou D2 ou ledit mélange M2 ayant une température inférieure à la température de la phase L1, lesdits moyens permettant d'introduire ladite phase L2 ou D2 ou ledit mélange M2 de façon à ce que leur écoulement ait lieu dans la même direction que l'écoulement du mélange M1 jusqu'à une première sortie interne (3'), de diamètre interne (Di) inférieur à (Dc), opposée à ladite première entrée interne (3), par laquelle ladite phase L2 ou ladite phase D2 ou ledit mélange M2 quitte par ladite sortie (3') ladite première enceinte intérieure,
- au moins une deuxième enceinte intérieure de forme allongée le long d'au moins un axe, de section sensiblement circulaire, disposée coaxialement par rapport à ladite première enceinte intérieure, comprenant une première extrémité située à une distance (Le) de la première sortie interne (3') de la première enceinte intérieure, ladite distance (Le) étant telle que la somme des distances (Le) et (Li) soit au plus égale à (L), dans laquelle pénètre, par une entrée (4) dite deuxième entrée interne, de diamètre interne (De) supérieur ou égal à (Di) et inférieur à (Dc), au moins une partie de la phase légère L1 et au moins une partie de la phase légère L2 ou de la phase dense D2 ou du mélange M2, ladite deuxième enceinte intérieure comprenant à une sortie (4') dite deuxième sortie interne située à proximité de la sortie externe (7) de l'enceinte extérieure, au moins un moyen d'évacuation permettant d'évacuer par ladite deuxième sortie interne, un mélange M3 comprenant au moins une partie de la phase légère L1 et au moins une partie de la phase légère L2 ou de la phase dense D2 ou du mélange M2, et
- au moins un moyen permettant de récupérer à partir du mélange M3 d'une part ladite phase L2 ou ladite phase D2 ou ledit mélange M2 et d'autre part la phase L1 refroidie contenant les hydrocarbures oléfiniques formés au cours de la conversion.

**Claims**

1. Process for the catalytic conversion of a charge comprising at least one oxygen compound, into olefinic hydrocarbons rich in compounds having 2 to 4 carbon atoms in their molecule and comprising the entrained bed conversion of said charge, in an elongated conversion reaction zone, under appropriate conditions and in the presence of a catalyst in the form of solid particles, said process including:
    - a) a supply stage to a zone in the vicinity of a first end of said reaction zone of at least one dense phase D1 in the form of solid particles containing catalytic particles,
    - b) a supply stage, in a zone located in the vicinity of a first end of said reaction zone of at least one entrainment fluid,
    - c) an introduction stage and, in the case of an at least partly liquid charge, the atomization of said charge in an introduction zone located downstream, in the solid particle displacement direction, with respect to the introduction zone of said solid particles,
    - d) a stage of contacting said solid particles and said charge in a zone located in the vicinity of the first end,
    - e) a stage of circulating the solid particles and the charge in the conversion zone during which the conversion of said charge takes place and the catalytic solid particles are at least partly deactivated by the deposition of coke thereon,

- f) a separation stage, from their mixture M1, of the dense phase D1 and the gases, forming a light phase L1, containing the products of the at least partial conversion of said charge in a separation and quenching zone located in the vicinity of a second end of the conversion zone opposite to said first end,

- g) a stage of regenerating, in at least one regenerating zone, at least part of the solid catalytic particles contained in the dense phase D1 and which are at least partly deactivated and

- h) a stage of recycling said dense phase D1 containing the solid catalytic particles in an at least partly regenerated form, into a recycling zone in the vicinity of said first end,

characterized in that during stage f), the dense phase D1 is separated from the light phase L1 containing the conversion products and said light phase is quenched in a separation and quenching zone incorporating in combination:

- at least one external enclosure, extended along at least one axis, having a substantially circular cross-section of diameter (Dc) and length (L), into which is introduced in the vicinity of a first end of said external enclosure by an intake (1), called the external intake, the mixture M1 which is circulated from said first end to a second end opposite to the first end of said external enclosure, whilst giving at least to the phase L1 of said mixture a helical movement in the flow direction of said mixture M1, the dense phase D1 is separated from the light phase L1, by means of an outlet (7), called the external outlet, recovery takes place of at least part of the dense phase D1 in the vicinity of said second end and the light phase L1 is passed into the intake (4), called the second internal intake, of the second internal enclosure described hereinafter,

- at least one first internal enclosure, extended along at least one axis and having a substantially circular cross-section, which has a length (Li) smaller than (L) and arranged coaxially relative to said external enclosure, into which is introduced by an intake (3), called the first internal intake, in the vicinity of the external intake of the external enclosure, a light phase L2 or a dense mixture D2 or a mixture M2 comprising both the light phase L2 and a dense phase D2, said phases L2 or D2 or said mixture M2 having a temperature below the temperature of the phase L1, in the same direction as the mixture M1, the said phases L2 or D2 or said mixture M2 is circulated from said first internal intake to a first internal outlet (3'), which has an internal diameter (Di) smaller than (Dc), which is opposite to said first internal intake (3), by which the said phase L2 or the said phase D2 or the said mixture M2 leaves said first internal enclosure by said outlet (3'),

- at least one second internal enclosure extended along at least one axis having a substantially circular cross-section and positioned coaxially relative to said first internal enclosure, comprising a first end located at a distance (Le) from the first internal outlet (3') of the first internal enclosure, said distance (Le) being such that the sum of the distances (Le) and (Li) is at the most equal to (L) and into which penetrates, by an intake (4) called the second internal intake and having an internal diameter (De) equal to or larger than (Di) and smaller than (Dc), at least part of the light phase L1 and at least part of the light phase L2 or the dense phase D2 or the mixture M2, and from said second enclosure is discharged by a second internal outlet (4'), which is opposite to said second internal intake, a mixture M3 comprising at least part of the light phase L1 and at least part of the light phase L2 or the dense phase D2 or the mixture M2 and

- the mixture M3 is passed into a zone in which separation and recovery takes place on the one hand of said phase L2 or said phase D2 or said mixture M2 and on the other hand the cooled phase L1 containing the olefinic hydrocarbons formed during the conversion,

2. Process according to claim 1, wherein the distance (Le), between the first internal outlet (3') of the first internal enclosure and the first internal inlet (4) of the second internal enclosure, is approximately 0.1x(Dc) to approximately 10x(Dc).

3. Process according to claims 1 or 2, wherein the mixture M1 is introduced into the separation and quenching zone in a direction substantially perpendicular to the axis of its external enclosure, or in a direction substantially parallel to the axis of said external enclosure.

4. Process according to any one of the claims 1 to 3, wherein the separation and quenching zone comprises, in the space located between the outer wall of the second internal enclosure and the inner wall of the external enclosure, downstream of the second internal intake in the flow direction of the various phases, at least one means limiting the advance of the light phase L1.

5. Process according to claim 4, wherein the means limiting the advance of the light phase L1 in the space between the outer wall of the second internal enclosure and the inner wall of the external enclosure are substantially planar blades, whose plane includes the axis of said external enclosure.

6. Process according to claim 5, wherein the separation and quenching zone has 2 to approximately 50 blades fixed to the outer wall of the second internal enclosure, so that the distance between the second internal intake and the point of said blades closest to said second internal intake is approximately 0 to approximately 5x(Dc).

7. Process according to claims 5 or 6, wherein the blades have in each case a dimension (ep), measured in the direction perpendicular to the axis of the external enclosure of said separation and quenching zone, of approximately 0.01 to approximately 1 times the value [((Dc)-(D'e))/2] corresponding to the distance between the outer wall of the second internal enclosure of external diameter (D'e) and the internal wall of the external enclosure of internal diameter (Dc), a dimension (hpe), measured on the edge of the blade closest to the axis of the internal enclosures in the direction parallel to said axis, and a dimension (hpe), measured in the direction parallel to the axis of the external enclosure of said separation and quenching zone on the edge of the blade closest to the internal wall of the external enclosure, said dimensions (hpi) and (hpe) being approximately 0.1x(Dc) to approximately 10x-(Dc).

8. Process according to claim 7, wherein the blades have in each case a dimension (hpi) equal to or greater than(hpe).

9. Process according to any one of the claims 1 to 8, wherein a fluid (L3) making it possible to strip the solid particles with respect to the dense phase D1 is introduced into the separation and quenching zone.

10. Process according to any one of the claims 1 to 9, wherein the charge comprises at least one alcohol, preferably methanol and wherein it is introduced in the gaseous state.

11. Apparatus for the entrained bed catalytic conversion of a charge containing at least one oxygen compound into olefinic hydrocarbons rich in compounds having 2 to 4 carbon atoms in their molecule, having an elongated enclosure, in which the said conversion is performed under appropriate conditions and comprising, in the vicinity of a first end, from upstream to downstream in the charge displacement direction, at least one means for introducing at least one entrainment fluid, at least one means for introducing at least one solid forming a dense phase D1 containing catalytic particles, at least one means for introducing the said charge, the apparatus comprising in the vicinity of a second end of said enclosure at least one means, connected to said enclosure, for separating from their mixture M1 solid particles and gases forming a light phase L1 and the quenching of said gases containing the products of the at least partial conversion of said charge and at least one means for regenerating at least part of the catalytic solid particles connected on the one hand to the said separation and quenching means and on the other to the said enclosure in the vicinity of said first end, so as to permit the at least partial regeneration of the solid catalytic particles and the recycling of said solid particles in said enclosure, characterized in that the means for separating the solid particles and for quenching the gases is a cocurrent cyclone separator - mixer comprising in combination:

- at least one external enclosure, extended along at least one axis, having a substantially circular cross-section of diameter (Dc) and length (L), having in the vicinity of a first end at least one introduction means permitting the introduction, by an intake (1) called the external intake, of the mixture M1 containing the dense phase D1 and the light phase L1, said means being able to give at least to the phase L1 a helical movement in the flow direction of said mixture M1 into the external enclosure and also having means for separating the phases D1 and L1 and, in the vicinity of a second end opposite to the first end, recovery means making it possible to recover by an outlet (7), called the external outlet, at least part of the dense phase D1,

- at least one first internal enclosure, extended along at least one axis, having a substantially circular cross-section and having a length (Li) smaller than (L) and arranged coaxially with respect to said external enclosure, having an intake (3), called the first internal intake, located in the vicinity of the external intake of the external enclosure, at least one introduction means making it possible to introduce by said first internal intake a light phase L2 or a dense phase D2 or a mixture M2 incorporating both a light phase L2 and a dense phase D2, said phases L2 or D2 or said mixture M2 having a temperature below the temperature of the phase L1, said means making it possible to introduce said phase L2 or D2 or said mixture M2 in such a way that their flow takes place in the same direction as the flow of the mixture M1 up to a first internal outlet (3') having an internal diameter (Di) smaller than (Dc), opposite to said first internal intake (3), by which said phase L2 or said phase D2 or said mixture M2 leaves by the said outlet (3') the said first internal enclosure,

- at least one second internal enclosure, extended along at least one axis, having a substantially circular cross-section and positioned coaxially with respect to the first internal enclosure and having a first end located at a distance (Le) from the first internal inlet (3') of the first internal enclosure, said distance (Le) being such that the sum of the distances (Le) and (Li) is at the most equal to (L) and which is entered, via on intake (4), called the second internal intake, of internal diameter (De) equal to or larger than (Di) and smaller than (Dc), at least part of the light phase L1 and at least part of the light phase L2 or the dense phase D2 or the mixture M2, said second internal enclosure having an outlet (4'), called the second internal outlet, located in the vicinity of the external outlet (7) of the external enclosure, at least one discharge means permitting the discharge by said second internal outlet of a mixture M3 comprising at least part of the light phase L1 and at least part of the light phase L2 or the dense phase D2 or the mixture M2 and

- at least one means making it possible to separate and recover from said mixture M3 on the one hand the said phase L2 or the said phase D2 or the said mixture M2 and on the other the cooled phase L1 containing the olefinic hydrocarbons formed during the conversion.

**Patentansprüche**

1. Verfahren zur katalytischen Umwandlung einer wenigstens eine sauerstoffhaltige Verbindung enthaltenden Charge in olefinische Kohlenwasserstoffe, die reich an Verbindungen mit 2 bis 4 Kohlenstoffatomen in ihrem Molekül sind, die Umwandlung im Schleppbett dieser Charge in einer Umwandlungsreaktionszone länglicher Gestalt unter geeigneten Bedinungen, in Anwesenheit eines Katalysators in Form von Feststoffpartikeln, umfassend, wobei dieses Verfahren umfaßt:

- a) Eine Stufe bei der in eine benachbart einem ersten Ende dieser Reaktionszone wenigstens eine dichte Phase D1 in Form von katalytische Partikel enthaltenden Feststoffpartikeln eingespeist wird,

- b) eine Stufe, bei der in eine benachbart einem ersten Ende dieser Reaktionszone angeordnete Zone wenigstens ein Schleppfluid eingespeist wird,

- c) eine Stufe, bei der diese Charge in eine Einführungszone stromabwärts in Bewegungsrichtung der Feststoffpartikel zur Einführungszone dieser Partikel eingeführt und im Falle einer wenigstens teilweise flüssigen Charge versprüht wird,

- d) eine Stufe, bei der diese Feststoffpartikel und diese Charge in einer benachbart dem ersten Ende befindlichen Zone kontaktiert werden,

- e) eine Stufe, bei der Feststoffpartikel und Charge in der Umwandlungszone in Zirkulation versetzt werden, während der man die Umwandlung dieser Charge vornimmt und man wenigstens zum Teil die katalytischen Feststoffpartikel durch Abscheiden von Koks hierauf desaktiviert,

- f) eine Stufe, bei der wenigstens teilweise ausgehend von ihrem Gemisch M1 der dichten Phase D1 und der eine leichte Phase L1 bildenden Gase, welche die Umwandlungsprodukte enthalten, diese Charge in einer Trenn- und Abschreckzone getrennt wird, die benachbart einem zweiten Ende dieser Umwandlungszone, die dem ersten Ende gegenüberliegt, angeordnet ist,

- g) eine Stufe, bei der in wenigstens einer Regenerationszone wenigstens ein Teil der katalytischen in der dichten Phase D1 befindlichen wenigstens zum Teil desaktivierten Partikel regeneriert werden und

- h) eine Stufe der Rezyklierung dieser dichten die katalytischen festen wenigstens zum Teil regenerierten Partikel enthaltenden Phase D1 in eine Rezyklierungszone benachbart diesem ersten Ende rezykliert bzw. rückgeführt werden,

**dadurch gekennzeichnet**, daß während der Stufe f) man die dichte Phase D1 von der leichten die Umwandlungsprodukte enthaltenden Phase L1 trennt und die Abschreckung dieser leichten Phase in einer Trenn- und Abschreckzone vornimmt, in Kombination umfassend:

wenigstens einen äußeren umschlossenen Raum von längs wenigstens einer Achse länglicher Gestalt, von einem im wesentlichen kreisförmigen Querschnitt vom Durchmesser (Dc) und von der Länge (L), in den man benachbart einem ersten Ende dieses äußeren umschlossenen Raums über einen Eingang (1), den sogenannten äußeren Eingang das Gemisch M1 einführt, das man ausgehend von diesem ersten Ende gegen ein diesem ersten Ende gegenüberliegendes zweites Ende dieses äußeren umschlossenen Raums zirkulieren läßt, indem man der Phase L1 dieses Gemisches eine spiralförmige Bewegung in Strömungsrichtung dieses Gemisches M1 erteilt, man die dichte Phase D1 von der leichten Phase L1 trennt, über einen Ausgang (7), den sogenannten äußeren Ausgang wenigstens einen Teil der dichten Phase D1 benachbart dem zweiten Ende gewinnt und die leichte Phase L1 in den Eintritt (4), den sogenannten zweiten inneren Eingang des zweiten inneren umschlossenen, im folgenden inneren umschlossenen Raumes genannten Raumes, schickt,

- wenigstens einen ersten inneren umschlossenen Raum von längs wenigstens einer Achse länglicher Gestalt von im wesentlichen kreisförmigem Querschnitt von der Länge (Li) kleiner als (L), der koaxial bezogen auf diesen äußeren umschlossenen Raum angeordnet ist, in den man über einen Eintritt (3), den sogenannten ersten inneren Eintritt benachbart dem äußeren Ende des äußeren umschlossenen Raumes, eine leichte Phase L2 oder eine dichte Phase D2 oder ein Gemisch M2 einführt, das gleichzeitig eine leichte Phase L2 und eine dichte Phase D2 umfaßt, wobei diesen Phasen L2 oder D2 oder dieses Gemisch M2 eine Temperatur geringer als die Temperatur der Phase L1 hat, man in der gleichen Richtung wie das Gemisch M1 diese Phasen L2 oder D2 oder dieses Gemisch M2 ausgehend von diesem ersten inneren Eingang bis zu einem ersten inneren Ausgang von einem Innendurchmesser (Di) kleiner als (Dc) schickt, der diesem ersten inneren Eingang (3) gegenüberliegt, über welchen diese Phase L2 oder diese Phase D2 oder dieses Gemisch M2 über den Ausgang (3') diesen ersten inneren umschlossenen Raum verläßt,

- wenigstens einen zweiten inneren umschlossenen Raum von einer längs wenigstens einer Achse länglichen Gestalt und einem im wesentlichen kreisförmigen Querschnitt, der koaxial bezüglich dieses ersten umschlossenen inneren Raums angeordnet ist und ein erstes Ende umfaßt, das unter einer Entfernung (Le) von diesem ersten inneren Ausgang (3') des ersten inneren umschlossenen Raums angeordnet ist, wobei diese Entfernung (Le) derart ist, daß die Summe der Entfernung (Le) und (Li) höchstens gleich (L) wird, in den über einen Eingang (4) den sogenannten zweiten inneren Eingang vom Innendurchmesser (De) größer oder gleich (Di) und kleiner (Dc) wenigstens ein Teil der leichten Phase L1 und wenigstens ein Teil der leichten Phase L2 oder der dichten Phase D2 oder des Gemisches M2 eindringt und man diesen zweiten umschlossenen Raum über einen zweiten inneren Ausgang (4'), der diesem zweiten inneren Eingang gegenüberliegt, ein Gemisch M3 abzieht, das wenigstens zum Teil einen Teil der leichten Phase L1 und wenigstens einen Teil der leichten Phase L2 oder der dichten Phase D2 oder des Gemisches M2 abzieht und

- man das Gemisch M3 in eine Zone schickt, in der man einerseits dieses Phase L2 oder diese Phase D2 oder dieses Gemisch M2 und andererseits die gekühlte olefinische während der Umwandlung gebildete Kohlenwasserstoffe enthaltende Phase trennt und gewinnt.

2. Verfahren nach Anspruch 1, bei dem der Abstand (Le) zwischen dem ersten inneren Ausgang (3') des ersten inneren umschlossenen Raums und dem zweiten inneren Eingang (4) des zweiten inneren umschlossenen Raums etwa 0,1x(Dc) bis etwa 10x(Dc) beträgt.

3. Verfahren nach Anspruch 1 oder 2, bei dem das Gemisch M1 in die Trenn- und Abschreckzone gemäß einer Richtung im wesentlichen senkrecht zur Achse ihres äußeren umschlossenen Raums oder gemäß einer Richtung im wesentlichen parallel zur Achse dieses äußeren umschlossenen Raums eingeführt wird.

4. Verfahren nach einem der Ansprüche 1 bis 3, bei dem die Trenn- und Abschreckzone in dem Raum zwischen der Außenwand des zweiten umschlossenen inneren Raums und der inneren Wandung des äußeren umschlossenen Raums hinter dem zweiten inneren Eingang in Strömungsrichtung der verschiedenen Phasen gesehen, wenigstens ein Mittel umfaßt, das die fortschreitende Bewegung der leichten Phase L1 begrenzt.

5. Verfahren nach Anspruch 4, bei dem das oder die die fortschreitende Bewegung der leichten Phase L1 in den Raum zwischen der Außenwandung des zweiten inneren umschlossenen Raums und der Innenwandung des äußeren umschlossenen Raums begrenzenden Mittel, im wesentlichen plane Schaufeln sind, deren Ebene die Achse dieses äußeren umschlossenen Raums umfaßt.

6. Verfahren nach Anspruch 5, bei dem die Trenn- und Kühlzone 2 bis etwa 50 Schaufeln umfaßt, die an der Außenwandung des zweiten inneren umschlossenen Raums derart befestigt sind, daß der Abstand zwischen dem zweiten Inneneingang und dem Punkt dieser Schaufeln, der diesem zweiten Inneneingang am weitesten benachbart ist, etwa 0 bis etwa 5x(Dc) beträgt.

7. Verfahren nach Anspruch 5 oder 6, bei dem die Schaufeln je eine Abmessung (ep), gemessen in der Richtung senkrecht zur Achse des äußeren umschlossenen Raums dieser Trenn- und Abschreckzone vom etwa 0,01 bis etwa dem 1-fachen des Wertes [((Dc)-(De))/2] entsprechend der Entfernung zwischen der Außenwand des zweiten umschlossenen Innenraums vom Außendurchmesser (De) und der Innenwandung des äußeren umschlossenen Raums vom Durchmesser (Dc), eine Abmessung (hpi), gemessen über die Kante der Schaufel, die der Achse der inneren umschlossenen Räume in der Richtung parallel zu dieser Achse am weitesten benachbart ist sowie eine Abmessung (hpe) haben, gemessen in der Richtung parallel zur Achse des äußeren umschlossenen Raums dieser Trenn- und Abschreckzone über die Kante der Schaufel, die der Innenwandung des äußeren umschlossenen Raums am weitesten benachbart ist, wobei diese Abmessung (hpi) und (hpe) etwa 0,1x-(Dc) bis etwa 10x(Dc) betragen.

8. Verfahren nach Anspruch 7, bei dem die Schaufeln je eine Abmessung (hpi) größer oder gleich (hpe) haben.

9. Verfahren nach einem der Ansprüche 1 oder 8, bei dem man in die Trenn- und Abschreckzone wenigstens ein Fluid (L3) einführt, das es ermöglicht, das Strippen der Feststoffpartikel von der dichten Phase D1 durchzuführen.

10. Verfahren nach einem der Ansprüche 1 bis 10, bei dem die Charge wenigstens einen Alkohol bevorzugt Methanol umfaßt, und bei dem sie im gasförmigen Zustand eingeführt wird.

11. Vorrichtung zur katalytischen Umwandlung, im Schleppbett, einer wenigstens eine sauerstoffhaltige Verbindung enthaltenden Charge in olefinische Kohlenwasserstoffe, die reich an Verbindungen mit 2 bis 4 Kohlenstoffatomen in ihrem Molekül sind, einen umschlossenen Raum länglicher Gestalt umfassend, in dem diese Umwandlung unter geeigneten Bedingungen vorgenommen wird, der benachbart einem ersten Ende von der Anströmseite zur Abströmseite in Bewegungsrichtung der Charge gesehen, wenigstens ein Mittel zum Einführen wenigstens eines Schleppfluids, wenigstens ein Mittel zum Einführen wenigstens eines eine dichte katalytische Partikel enthaltende Phase D1 bildenden Feststoff, wenigstens ein Mittel zum Einführen dieser Charge umfaßt, wobei diese Vorrichtung benachbart einem zweiten Ende dieses umschlossenen Raums wenigstens ein mit diesem umschlossenen Raum verbundendes Mittel zum Trennen, ausgehend von ihrem Gemisch M1, der Feststoffpartikel und der eine leichte Phase L1 bildenden Gase und zum Abkühlen dieser Gase umfaßt, welche die Produkte der wenigstens teilweisen Umwandlung dieser Charge enthält und wenigstens ein Mittel zum Regenerieren wenigstens eines Teils der katalytischen Feststoffpartikel, die einerseits mit diesem Trenn- und Abschreckmittel und andererseits mit diesem umschlossenen Raum benachbart diesem ersten Ende derart verbunden ist, daß die Regenerierung wenigstens eines Teils der katalytischen Feststoffpartikel und der Rezyklierung dieser Feststoffpartikel in diesem umschlossenen Raum möglich gemacht wird, dadurch gekennzeichnet, daß dieses Mittel zum Trennen der Feststoffpartikel und zur Abschreckung der Gase ein Mischer-Gleichstrom-Zyklonseparator ist,

in Kombination umfassend:

- wenigstens einen äußeren umschlossenen Raum von längs wenigstens einer Achse länglicher Gestalt mit einem im wesentlichen kreisförmigen Querschnitt vom Durchmesser (Dc) und der Länge (L), benachbart einem ersten Ende wenigstens ein Einführungsmittel umfassend, das es ermöglicht, über einen Eingang (1), den sogenannten äußeren Eingang, das Gemisch M1 einzuführen, das die dichte Phase D1 und die leichte Phase L1 enthält, wobei dieses Mittel so ausgelegt ist, daß es wenigstens der Phase L1 eine spiralförmige Bewegung in der Strömungsrichtung dieses Gemisches M1 in diesem äußeren umschlossenen Raum erteilt und ebenfalls Trennmittel für die Phasen D1 und L1 und benachbart einem zweiten dem ersten Ende gegenüberliegenden Ende Gewinnungsmittel umfaßt, die es ermöglichen, über einen Ausgang (7), den sogenannten äußeren Ausgang, wenigstens einen Teil der dichten Phase D1 rückzugewinnen,

- wenigstens einen ersten inneren umschlossenen Raum von längs wenigstens einer Achse länglicher Gestalt von im wesentlichen kreisförmigem Querschnitt von der Länge (Li) kleiner (L), der koaxial bezogen auf diesen äußeren umschlossenen Raum angeordnet ist, der an einem Eingang (3) dem sogenannten inneren Eingang, der benachbart dem äußeren Eingang dieses äußeren umschlossenen Raums angeordnet ist wenigstens ein Einführungsmittel umfaßt, das es ermöglicht, über diesen ersten inneren Eingang eine leichte Phase L2 oder eine dichte Phase D2 oder ein Gemisch M2 einzuführen, das gleichzeitig eine leichte Phase L2 und eine dichte Phase D2 umfaßt, wobei diese Phasen L2 oder D2 oder dieses Gemisch M2 eine Temperatur kleiner als die Temperatur der Phase L1 hat, wobei diese Mittel es ermöglichen, diese Phase L2 oder D2 oder dieses Gemisch M2 derart einzuführen, daß deren Strömung in der gleichen Richtung wie die Strömung des Gemisches M1 bis zu einem ersten inneren Ausgang (3') von einem Durchmesser (Di) kleiner als (Dc) stattfindet, der diesem ersten inneren Eingang (3) gegenüberliegt, über den diese Phase L2 oder diese Phase D2 oder dieses Gemisch M2 über diesen Ausgang (3') diesen ersten inneren umschlossen Raum verlassen,

- wenigstens einen zweiten inneren umschlossenen Raum von längs wenigstens einer Achse länglicher Gestalt von im wesentlichen kreisförmigem Querschnitt, der koaxial bezüglich dieses ersten inneren umschlossenen Raums angeordnet ist und ein erstes Ende umfaßt, das unter einer Entfernung (Le) vom ersten inneren Ausgang (3') des ersten inneren umschlossenen Raums angeordnet ist, wobei der Abstand (Le) derart ist, daß die Summe der Entfernungen (Le) und (Li) höchstens gleich (L) wird, in den über einen Eingang (4), den sogenannten zweiten inneren Eingang vom Innendurchmesser (De) größer oder gleich (Di) und kleiner (Dc) wenigstens ein Teil der leichten Phase L1 und wenigstens ein Teil der leichten Phase L2 oder der dichten Phase D2 oder des Gemisches M2 eindringt, wobei diese zweite Innenkammer an einem Ausgang (4') dem sogenannten zweiten Innenausgang, der benachbart dem äußeren Ausgang (7) des äußeren umschlossenen Raums angeordnet ist, wenigstens ein Abzugsmittel umfaßt, das es ermöglicht, über diesen zweiten Innenausgang ein Gemisch M3 abzuziehen, das wenigstens einen Teil der leichten Phase L1 und wenigstens einen Teil der leichten Phase L2 oder der dichten Phase D2 oder des Gemisches M2 umfaßt, und

- wenigstens ein Mittel, das es ermöglicht, ausgehend vom Gemisch M3 einerseits diese Phase L2 oder diese Phase D2 oder dieses Gemisch M2 und andererseits die gekühlte Phase L1 zu gewinnen, welche die während der Umwandlung gebildeten olefinischen Kohlenwasserstoffe enthält.

**FIG.2A**

**FIG.2B**

**FIG.1**

19

## FIG.3

## FIG.4